(19) Europäisches Patentamt
European Patent Office
Office européen des brevets

(11) EP 1 719 764 A1

(12) EUROPEAN PATENT APPLICATION
published in accordance with Art. 158(3) EPC

(43) Date of publication:
**08.11.2006 Bulletin 2006/45**

(21) Application number: **05719508.3**

(22) Date of filing: **25.02.2005**

(51) Int Cl.:
***C07D 233/60*** (2006.01) ***C07B 53/00*** (2006.01)
*C07B 61/00* (2006.01)

(86) International application number:
**PCT/JP2005/003107**

(87) International publication number:
**WO 2005/082861 (09.09.2005 Gazette 2005/36)**

(84) Designated Contracting States:
**AT BE BG CH CY CZ DE DK EE ES FI FR GB GR HU IE IS IT LI LT LU MC NL PL PT RO SE SI SK TR**

(30) Priority: **27.02.2004 JP 2004054928**

(71) Applicant: **Mitsubishi Pharma Corporation**
**Osaka-shi,**
**Osaka 541-0046 (JP)**

(72) Inventor: **USHIO, Hiroyuki,**
**c/o Mitsubishi Pharma Corporation**
**Chuo-ku, Tokyo 1038405 (JP)**

(74) Representative: **von Kreisler, Alek et al**
**Deichmannhaus am Dom,**
**Postfach 10 22 41**
**50462 Köln (DE)**

# (54) METHOD OF ASYMMETRICALLY REDUCING 4- 5-(IMIDAZOL-1-YL)-2-MET HYLBENZOYL -3,5-DIMETHYL-BENZOIC ACID OR ESTER THEREOF

(57) The invention relates to a method of producing an optically active 4-[hydroxy[5-(imidazol-1-yl)-2-methylphenyl]methyl]-3,5-dimethylbenzoic acid or an ester thereof, which includes reacting 4-[5-(imidazol-1-yl)-2-methylbenzoyl]-3,5-dimethylbenzoic acid or an ester thereof with a silane agent in the presence of a particular zinc compound and an optically active diamine compound.



Printed by Jouve, 75001 PARIS (FR)

## Description

### Technical Field

The present invention relates to a method of preparing an optically active 4-[hydroxy[5-(imidazol-1-yl)-2-methylphenyl]methyl]-3,5-dimethylbenzoic acid or an ester thereof from 4-[5-(imidazol-1-yl)-2-methylbenzoyl]-3,5-dimethylbenzoic acid or an ester thereof.

### Background Art

A composition comprising, as an active ingredient, 4-[hydroxy[5-(imidazol-1-yl)-2-methylphenyl]methyl]-3,5-dimethylbenzoic acid represented by the following structural formula:
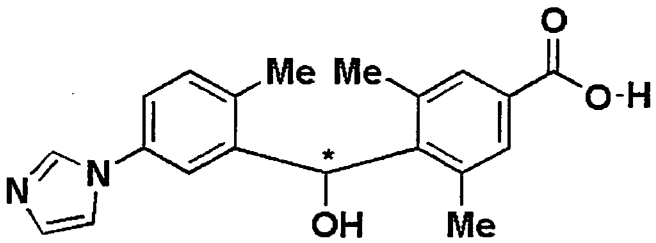
,

an optically active form thereof or a pharmaceutically acceptable salts thereof are useful for the prophylaxis or the treatments of diabetic neuropathy, nephropathy, ocular disorder and arteriosclerosis (see patent reference 1). In addition, a method of optical resolution of a racemate via their diastereomer esters is known (see patent reference 2).
As methods for asymmetric reduction of carbonyl group of a benzophenone derivatives having substituent(s), hydrogen transfer reduction of 4-cyano-4'-methoxybenzophenone in the presence of ruthenium catalysts (see non-patent reference 1), a hydrogen addition reaction in the presence of ruthenium-BINAP catalysts (see non-patent reference 2), and a method comprising use of a boron hydride reagent or an aluminum hydride reagent, having an optically active amino alcohol derivative as a ligand (non-patent reference 3) are known. However, a method for efficient asymmetric reduction of a carbonyl group of a derivative having high steric hindrance due to plural substituents at the ortho positions, such as a benzophenone derivative represented by the following formula (I)
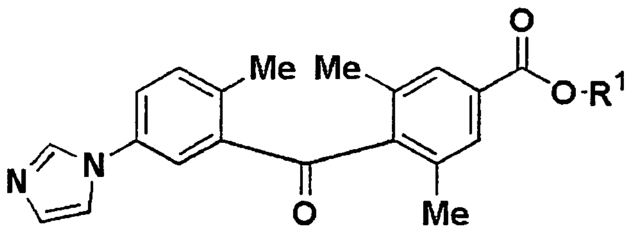
(I)

wherein $COOR^1$ is a carboxylic acid or a carboxylate, having substituent(s) at the 2- and 6-positions, and 2'-position, has not been known.
In recent years, ionic hydrogenation using a silane agent such as silane, siloxane and the like as a hydrogen source has been drawing attention (see non-patent reference 4). In this hydrosilylation reaction, asymmetric hydrosilylation reaction can be carried out under mild conditions where metal hydride is produced in a reaction system in the co-presence of an asymmetric metal complex containing copper, titanium and the like as a central metal (see non-patent references 5-7). However, when the asymmetric hydrosilylation reaction is applied to a compound having a carbonyl group and a carboxyl group or an alkoxycarbonyl group in a molecule, like a compound represented by the formula (I), not only the carbonyl group but also the carboxyl group and the alkoxycarbonyl group may be reduced (see non-patent reference 5).

Recently, H. Mimoun et al. have reported an asymmetric reduction of a carbonyl group using a silane agent such as polymethylhydrosiloxane (PMHS) and the like in the presence of a zinc catalyst having optically active diamine as a ligand (see non-patent references 8, 9, 10 and patent reference 3). However, no reference has reported on an asymmetric reduction of a carbonyl group of a derivative having a high steric hindrance, such as a benzophenone derivative having substituent(s), particularly, benzophenone derivatives having plural substituents at the ortho positions.
[patent reference 1] WO 97/24333
[patent reference 2] JP-A-2000-290259
[non-patent reference 1] J. Am. Chem. Soc., 118, 2521-2522 (1996)
[non-patent reference 2] Org. Lett., 2, 659-662, (2000)
[non-patent reference 3] Angewandte Chemie International Edition in English, 37, 1986-2012, (1998)
[non-patent reference 4] Synthesis, 633-651, (1974)
[non-patent reference 5] J. Am. Chem. Soc., 121, 9473-9474, (1999)
[non-patent reference 6] Journal of Organometallic Chemistry, 624, 367-371, (2001)
[non-patent reference 7] J. Am. Chem. Soc., 123, 12917-12918, 2001
[non-patent reference 8] J. Am. Chem. Soc., 121, 6156-6166, 1999
[non-patent reference 9] J. Org. Chem., 64, 2582-2589, 1999
[non-patent reference 10] Tetrahedron, 60, 1781-1789, 2004
[patent reference 3] JP-A-2001-515875

## Disclosure of the Invention

### Problems to be Solved by the Invention

The present invention aims at providing a method for the asymmetric reduction of 4-[5-(imidazol- 1-yl)-2-methylbenzoyl]-3,5-dimethylbenzoic acid or an ester thereof efficiently to optically active 4-[hydroxy[5-(imidazol-1-yl)-2-methylphenyl]methyl]-3,5-dimethylbenzoic acid or an ester thereof.
The present inventors have conducted intensive studies in view of the above-mentioned problems and found that an asymmetric reduction proceeds efficiently by reacting 4-[5-(imidazol-1-yl)-2-methylbenzoyl]-3,5-dimethylbenzoic acid or an ester thereof with a silane agent in the presence of a particular zinc compound and a particular optically active diamine compound, which resulted in the completion of the present invention.
Accordingly, the present invention provides the following.

(1) A method of preparing optically active 4-[hydroxy[5-(imidazol-1-yl)-2-methylphenyl]methyl]-3,5-dimethylbenzoic acid represented by the following formula (II)

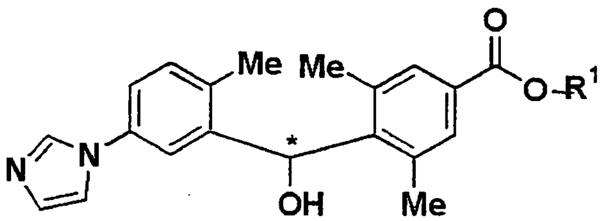

(II)

wherein $COOR^1$ is a carboxylic acid or a carboxylate and the carbon atom with * is an asymmetric carbon atom, or an ester thereof, from 4-[5-(imidazol-1-yl)-2-methylbenzoyl]-3,5-dimethylbenzoic acid represented by the following formula (I)
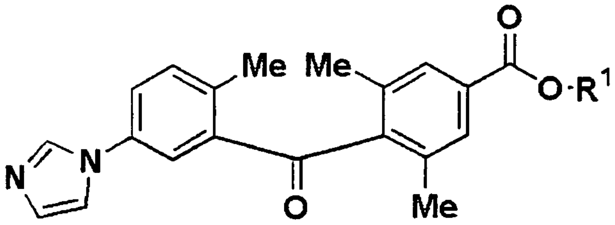

(I)

wherein $COOR^1$ is as defined above, or an ester thereof, which comprises reacting the compound represented by the formula (I) with a silane agent in the presence of a zinc compound represented by the following formula (III)
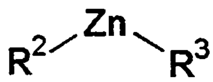

(III)

wherein $R^2$ and $R^3$ are each independently a lower alkyl group or a lower alkoxy group, or $R^2$ and $R^3$ in combination show an alkylenedioxy group optionally having substituent(s), and an optically active diamine compound represented by the following formula (IV)
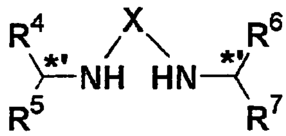

(IV)

wherein $R^4$ and $R^6$ are each independently a lower alkyl group, $R^5$ is an aryl group optionally having substituent (s), $R^7$ is an aryl group optionally having substituent(s) or a lower alkyl group, X is an alkylene group or a cycloalkylene group, and one or both of the two carbon atoms with *' is(are) asymmetric carbon atom(s).

(2) The method of (1), wherein $R^1$ is an alkyl group optionally having substituent(s), a cycloalkyl group optionally having substituent(s) or an aralkyl group optionally having substituent(s) on the ring and/or the chain.
(3) The method of (1) or (2), wherein $R^2$ and $R^3$ are each independently a lower alkyl group.
(4) The method of (3), wherein the reaction is carried out in the presence of alcohol or glycol.
(5) The method of any one of (1) to (4), wherein the optically active diamine compound is a N,N'-bis-(1-phenylethyl) ethane-1,2-diamine compound represented by the following formula (V)

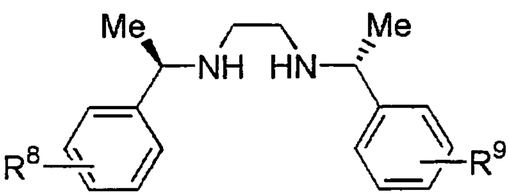

(V)

wherein $R^8$ and $R^9$ are each independently a hydrogen atom, a halogen atom, a lower alkyl group, a lower alkoxy group, a nitro group, a cyano group or an aryl group optionally having substituent (s),
or an optical isomer thereof.

(6) The method of (5), wherein the N,N'-bis-(1-phenylethyl)ethane-1,2-diamine compound is (R,R)-N,N'-bis-(1-phenylethyl)ethane-1,2-diamine or (R,R)-N,N'-bis-[1-(4-bromophenyl)ethyl]ethane-1,2-diamine.

(7) The method of any one of (1) to (6), wherein the silane agent is selected from the group consisting of trimethylsilane, diethylsilane, triethylsilane, phenylsilane, diphenylsilane, methylphenylsilane, dimethylphenylsilane, diethylphenylsilane, methyldiphenylsilane, tert-butyldimethylsilane, tert-butyldiphenylsilane, trimethoxysilane, diethoxysilane, triethoxysilane, tributoxysilane, triphenoxysilane, (trimethylsiloxy)dimethylsilane, bis(trimethylsiloxy)methylsilane, triisopropoxysilane, tris(trimethylsiloxy)silane, tris(trimethylsilyl)silane and polymethylhydrosiloxane.

(8) A method of preparing an optically active 4-[hydroxy[5-(imidazol-1-yl)-2-methylphenyl]methyl]-3,5-dimethylbenzoic acid ester which comprises reacting 4-[5-(imidazol-1-yl)-2-methylbenzoyl]-3,5-dimethylbenzoic acid ester with polymethylhydrosiloxane in the presence of zinc di-lower alkyl and an optically active diamine compound represented by the formula (V).

(9) The method of (8) further comprising a reaction in the presence of alcohol or glycol.

(10) The method of (9) further comprising a reaction in the presence of ether.

## Effect of the Invention

According to the method of the present invention, optically active 4-[hydroxy[5-(imidazol-1-yl)-2-methylphenyl]methyl]-3,5-dimethylbenzoic acid or an ester thereof can be produced high selectively in a high yield by asymmetric reduction of a carbonyl group of 4-[5-(imidazol-1-yl)-2-methylbenzoyl]-3,5-dimethylbenzoic acid or an ester thereof.

## Best Mode for Embodying the Invention

As $COOR^1$ of a compound represented by the formula (I) to be used in the present invention, carboxylate is preferable. As the alkyl group for $R^1$, for example, an alkyl group such as methyl, ethyl, propyl, isopropyl, butyl, isobutyl, sec-butyl, tert-butyl, pentyl, hexyl and heptyl groups and the like can be mentioned, with preference given to a linear or branched alkyl group having 1 to 7 carbon atoms.

As the substituent the alkyl group optionally has, for example, a halogen atom; the below-mentioned cycloalkyl group; the below-mentioned lower alkoxy group and the like can be mentioned.

As the cycloalkyl group, for example, a cycloalkyl group having 3 to 8 carbon atoms such as cyclopentyl and cyclohexyl groups and the like can be mentioned.

As the substituent the cycloalkyl group optionally has, for example, a halogen atom, a lower alkyl group and a lower alkoxy group to be mentioned below and the like can be mentioned.

As the aralkyl group, for example, benzyl, 1-phenylethyl, 2-phenylethyl, 3-phenylpropyl and naphthylmethyl group and the like can be mentioned.

As the substituent on the chain or the ring that the aralkyl group optionally has, for example, a halogen atom, a lower alkyl group, a lower alkoxy group and the like can be mentioned for each.

A compound of represented by the formula (I), wherein $COOR^1$ is carboxylate can be produced by subjecting 4-[hydroxy[5-(imidazol-1-yl)-2-methylphenyl]methyl]-3,5-dimethylbenzoic acid described in patent reference 2 to a known esterification reaction.

In the zinc compound, as the lower alkyl group for $R^2$ or $R^3$, for example, a linear or branched alkyl group having 1 to 4 carbon atoms, such as methyl, ethyl, propyl, isopropyl, butyl, isobutyl group and the like can be mentioned.

As the lower alkoxy group, for example, a linear or branched alkoxy group having 1 to 4 carbon atoms, such as methoxy, ethoxy, propoxy, isopropoxy, butoxy, isobutoxy group and the like can be mentioned.

The alkylenedioxy group may be linear or branched, and may have substituent(s), such as phenyl group and the like, on the chain. As the alkylenedioxy group, for example, ethylenedioxy, propylenedioxy, trimethylenedioxy, tetramethylenedioxy, pentamethylenedioxy, 1,3-dimethyltrimethylenedioxy, 1,2-cyclohexanedioxy, 1,2-diphenylethylenedioxy group and the like can be mentioned.

As the zinc compound, a zinc di-lower alkyl compound is preferable and, for example, dimethyl zinc, diethyl zinc and the like can be mentioned.

In the optically active diamine compound, as the lower alkyl group for $R^4$, $R^6$ or $R^7$, those recited for $R^2$ or $R^3$ of the aforementioned zinc compound can be mentioned. The two carbon atoms with *' are both preferably asymmetric carbons.

As the aryl group for $R^5$ or $R^7$, for example, phenyl and naphthyl groups and the like can be mentioned.

As the substituent the aryl group optionally has, for example, a halogen atom, a lower alkyl group, a lower alkoxy group and the like can be mentioned.

As the alkylene group, for example, an alkylene group having 2 to 5 carbon atoms can be mentioned, with preference given to an ethylene group or a trimethylene group.

As the cycloalkylene group, for example, a 1,2-cycloalkylene group having 4 to 8 carbon atoms such as a 1,2-cyclopentylene group or a 1,2-cyclohexylene group is preferable.

The optically active diamine compound can be produced by a known method, such as the methods described in Tetrahedron: Asymmetry, 14, 3937-3941 (2003); Tetrahedron: Asymmetry, 5, 699-708 (1994);, J. Am. Chem. Soc., 121, 6158-6166 (1999); Tetrahedron: Asymmetry, 5, 699-708, 1994; Tetrahedron: Asymmetry, 14, 3937-3941 (2003); Tetrahedron: Asymmetry, 5, 699-708 (1994); J. Am. Chem. Soc., 121, 6158-6166 (1999); J. C. S. Chem. Comm., 1409-1410 (1987); non-patent reference 10 and the like, or a method analogous thereto.

As the optically active diamine compound, a N,N'-bis-(1-phenylethyl)ethane-1,2-diamine compound represented by the following formula (V)

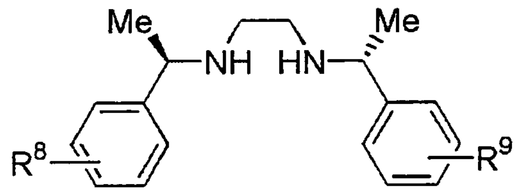

(V)

wherein $R^8$ and $R^9$ are each independently a hydrogen atom, a halogen atom, a lower alkyl group, a lower alkoxy group, a nitro group, a cyano group or an aryl group optionally having substituent(s), or an optical isomer thereof is preferable.

In the above-mentioned N,N'-bis-(1-phenylethyl)ethane-1,2-diamine compound, as the lower alkyl group or the lower alkoxy group for $R^8$ or $R^9$, those recited as $R^2$ or $R^3$ of the aforementioned zinc compound can be mentioned. As the aryl group, for example, a phenyl group, a naphthyl group and the like can be mentioned. As the substituent the aryl group optionally has, for example, a halogen atom, the aforementioned lower alkyl group, the aforementioned lower alkoxy group, a nitro group, a cyano group and the like can be mentioned.

As the N,N'-bis-(1-phenylethyl)ethane-1,2-diamine compound, for example, (R,R)-N,N'-bis-(1-phenylethyl) ethane-1,2-diamine, (R,R)-N,N'-bis-[1-(4-bromophenyl)ethyl]ethane-1,2-diamine, (S,S)-N,N'-bis-[1-(4-isobutylphenyl) ethyl]ethane-1,2-diamine, (S,S)-N,N'-bis-[1-(2-fluorobiphenyl-4-yl)ethyl]ethane-1,2-diamine, (S,S)-N,N'-bis-[1-(6-methoxynaphthalen-2-yl)ethyl]ethane-1,2-diamine, (R,R)-N,N'-bis-(1-naphthalen-1-ylethyl)ethane-1,2-diamine, (R,R)-N,N'-bis-[1-(4-nitrophenyl)ethyl]ethane-1,2-diamine, (R,R)-N,N'-bis-[1-(4-cyanophenyl)ethyl]ethane-1,2-diamine, (R,R)-N,N'-bis-[1-(4-methylphenyl)ethyl]ethane-1,2-diamine, (R,R)-N,N'-bis-[1-(4-tert-butylphenyl)ethyl]ethane-1,2-diamine, (R,R)-N,N'-bis-(1-phenylethyl)propane-1,3-diamine, (1R,2R,1'R,1''R)-N,N'-bis-(1-phenylethyl)cyclopentane-1,2-diamine, (R)-N-[1-(4-bromophenyl)ethyl]-N'-isopropylethane-1,2-diamine, (R)-N-benzyl-N'-(1-phenylethyl)ethane-1,2-diamine, (R,R)-N-[1-(4-bromophenyl)ethyl]-N'-(1-phenylethyl)ethane-1,2-diamine and (S,S)-N-[1-(4-isobutylphenyl) ethyl]-N'-(phenylethyl)ethane-1,2-diamine, and optical isomers thereof can be mentioned.

The silane agent to be used in the present invention is a silane compound or siloxane compound having a hydrogen atom on the silicon atom. As the silane compound, for example, trimethylsilane, diethylsilane, triethylsilane, phenylsilane, diphenylsilane, methylphenylsilane, dimethylphenylsilane, diethylphenylsilane, methyldiphenylsilane, tert-butyldimethylsilane, tert-butyldiphenylsilane, trimethoxysilane, diethoxysilane, triethoxysilane, tributoxy silane, triphenoxysilane and the like can be mentioned. As the siloxane compound, for example, (trimethylsiloxy)dimethylsilane, bis (trimethylsiloxy)methylsilane, triisopropoxysilane, tris(trimethylsiloxy)silane, tris(trimethylsilyl)silane, polymethylhydrosiloxane (PMHS) and the like can be mentioned.

The reaction is carried out by, after mixing a zinc compound with an optically active diamine compound, mixing 4-[5-(imidazol-1-yl)-2-methylbenzoyl]-3,5-dimethylbenzoic acid or an ester thereof with the mixture, and then mixing a silane agent therewith.

In general, not less than 0.001 mol of a zinc compound, not less than 0.001 mol of an optically active diamine compound, and not less than 1 mol of a silane agent are reacted, per 1 mol of 4-[5-(imidazol-1-yl)-2-methylbenzoyl]-3,5-dimethylbenzoic acid or an ester thereof. When the zinc compound is used in too small an amount, the reaction does not proceed easily, and therefore, not less than 0.01 mol thereof is preferably used. Use of a large amount thereof is meaningless and 1 mol thereof is sufficient. When the optically active diamine compound is used in too small an amount, the reaction does not proceed easily, and therefore, not less than 0.01 mol thereof is preferably used. Use of a large amount thereof is meaningless and 1 mol thereof is sufficient. When the amount the silane agent is small, the reaction does not proceed easily, and therefore, the silane agent is preferably used in an amount of not less than 1 mol. Use of a large amount thereof is meaningless and 10 mol thereof is sufficient. At this time, not less than 0.2 mol of the optically active diamine compound is preferably reacted, per 1 mol of the zinc compound. A greater ratio of the optically active diamine compound is meaningless and about 1 mol thereof is sufficient. When the zinc compound is zinc di-lower alkyl, the optically active diamine is preferably used in an almost equimolar amount.

The reaction solvent may be any as long as it does not inhibit the reaction. For example, aliphatic hydrocarbon such as n-hexane and the like; aromatic hydrocarbon such as toluene and the like; ether such as diethyl ether, 1,2-dimethoxyethane, 1,2-diethoxyethane, tetrahydrofuran, 1,4-dioxane and the like; aprotic polar solvent such as N,N'-dimethylformamide and the like; and the like can be mentioned. A mixed solvent of these may be used or different solvents may be used for each operation.

The reaction is preferably carried out in an inactive gas atmosphere such as nitrogen gas, argon gas and the like. The reaction temperature and reaction temperature can be appropriately determined depending on the kind and amount of each compound to be subjected to each reaction. A zinc compound and an optically active diamine compound can be mixed at a temperature of generally from 0°C to the boiling point of the reaction solvent, preferably at room temperature, for generally 1 minute to 12 hr, preferably about 10 minutes to 2 hr. 4-[5-(Imidazol-1-yl)-2-methylbenzoyl]-3,5-dimethylbenzoic acid or an ester thereof can be directly added, or after dissolving or suspending in a reaction solvent and mixed generally at a temperature of -10°C to 30°C, preferably at room temperature, for generally 1 minute to 1 hr, preferably about 5 minutes to 20 minutes. A silane agent can be directly added, or after dissolving or suspending in a reaction solvent, and mixed generally at a temperature of -10°C to 30°C, preferably room temperature, for generally 1 hr to 48 hr, preferably about 2 hr to 10 hr.

While the reaction is preferably carried out under anhydrous conditions, when water is present in the system, dehydrating agents such as molecular sieves (preferably MS3A, MS4A), anhydrous sodium sulfate, anhydrous calcium sulfate and the like may be added to the reaction mixture. To enhance selectivity and yield, an activated carbon may also be added.

When the zinc compound is di-lower alkyl zinc, the reaction is preferably carried out in the presence of alcohol or glycol since the yield can be increased. As the alcohol, alcohol having 1 to 4 carbon atoms, such as methanol, ethanol, propanol, isopropyl alcohol, butanol, isobutyl alcohol and the like can be mentioned. As the glycol, ethylene glycol, propylene glycol, trimethylene glycol, tetramethylene glycol, pentamethylene glycol, 1,3-dimethyltrimethylene glycol, cyclohexane-1,2-diol, 1,2-diphenylethylene glycol and the like can be mentioned. Not less than 1 mol and not more than 10 mol of alcohol, or not less than 0.5 mol and not more than 5 mol of glycol, per 1 mol of lower alkyl zinc, is preferably present in the reaction mixture. When the amount of alcohol is too small, the yield may decrease. Thus, alcohol is preferably used in an amount of not less than 1.5 mol. When its amount is too high, the selectivity may be degraded. Thus, alcohol is preferably used in an amount of not more than 6 mol. Like alcohol, when the amount of glycol is too small, the yield may decrease. Thus, glycol is preferably used in an amount of not less than 0.7 mol. Conversely, when its amount is too high, the selectivity may be degraded. Thus, alcohol is preferably used in an amount of not more than 3 mol.

When the reaction is carried out in the presence of alcohol or glycol, it is preferably carried out in the presence of a dehydrating agent such as molecular sieves and the like. It is more preferable to use, as a reaction solvent, ether such as diethyl ether, 1,2-dimethoxyethane, 1,2-diethoxyethane, tetrahydrofuran, 1,4-dioxane and the like, particularly tetrahydrofuran, alone or in combination with other solvent.

In general, for an asymmetric reduction of a carbonyl group in the case of high steric hindrance, such as the carbonyl group of a benzophenone derivative represented by the formula (I), the amount of the asymmetric catalyst to be used is increased to carry out the reaction quickly. However, when ether is used as a reaction solvent, the reduction reaction can be carried out highly selectively in a high yield with a small amount of an asymmetric catalyst. While the reason therefor is not certain, it is postulated that the asymmetric catalysts consisting of di-lower alkyl zinc, optically active diamine and alcohol or glycol are coordinated by the lone electron-pair of ether oxygen atoms to confer the catalyst with a structure suitable for the asymmetric reaction, which in turn stabilizes it.

The asymmetric reduction is quenched by adding a base such as sodium hydroxide solution and the like, an acid such as hydrochloric acid and the like or a fluoride such as potassium fluoride and the like to the reaction mixture. At that time, lower alcohol such as methanol, ethanol and the like may be added. Then the resulting solution is treated by a conventional method to give optically active 4-[hydroxy[5-(imidazol-1-yl)-2-methylphenyl]methyl]-3,5-dimethylbenzoic acid or esters thereof.

By hydrolyzing the obtained optically active 4-[hydroxy[5-(imidazol-1-yl)-2-methylphenyl]methyl]-3,5-dimethylbenzoic acid esters according to a conventional method, optically active 4-[hydroxy[5-(imidazol-1-yl)-2-methylphenyl]methyl]-3,5-dimethylbenzoic acid can be produced. When a base such as sodium hydroxide solution and the like is used after the completion of the above-mentioned reaction, optically active 4-[hydroxy[5-(imidazol-1-yl)-2-methylphenyl]methyl]-3,5-dimethylbenzoic acid can also be directly obtained without isolation of the optically active 4-[hydroxy[5-(imidazol-1-yl)-2-methylphenyl]methyl]-3,5-dimethylbenzoic acid esters.

## Examples

The present invention is explained in more detail in the following by referring to Examples, which are not to be construed as limitative.

The optical purity was calculated based on the value measured under the following HPLC conditions.
HPLC analysis conditions

column: YMC-Pack ODS-AM 150×6.0 mm I.D. (Pre)+CHIRALCEL OD-RH 15×0.46 cm I.D. (Post).
mobile phase: 0.05 mol/L aqueous sodium perchlorate solution (pH=2.0)/acetonitrile/methanol=12/4/1.
flow rate: 1.0 mL/minute
wave length: 243 nm
temperature: around 25°C
analysis time: about 65 minutes
retention time
compound 1: (S)-4-[hydroxy[5-(imidazol-1-yl)-2-methylphenyl]methyl]-3,5-dimethylbenzoic acid: about 19.5 minutes,
compound 2: (R)-4-[hydroxy[5-(imidazol-1-yl)-2-methylphenyl]methyl]-3,5-dimethylbenzoic acid: about 21.7 minutes,
compound 3: 4-[5-(imidazol-1-yl)-2-methylbenzoyl]-3,5-dimethylbenzoic acid: about 58.8 minutes

Optical purity and conversion
The optical purity and conversion rate shown below were calculated based on the following calculation formulas.
$$\text{optical purity} = \frac{\left(\begin{array}{c}\text{area percentage of}\\ \text{compound 1}\end{array}\right) - \left(\begin{array}{c}\text{area percentage of}\\ \text{compound 2}\end{array}\right)}{\left(\begin{array}{c}\text{area percentage of}\\ \text{compound 1}\end{array}\right) + \left(\begin{array}{c}\text{area percentage of}\\ \text{compound 2}\end{array}\right)}$$

$$\text{conversion} = \frac{\left(\begin{array}{c}\text{area percentage of}\\ \text{compound 1}\end{array}\right) + \left(\begin{array}{c}\text{area percentage of}\\ \text{compound 2}\end{array}\right)}{\left(\begin{array}{c}\text{area percentage}\\ \text{of compound 1}\end{array}\right) + \left(\begin{array}{c}\text{area percentage}\\ \text{of compound 2}\end{array}\right) + \left(\begin{array}{c}\text{area percentage}\\ \text{of compound 3}\end{array}\right)}$$

**Production Example 1**

(R,R)-N,N'-bis-[1-(4-bromophenyl)ethyl]ethane-1,2-diamine

(1) Under ice-cooling, to a solution (100 mL) of (R)-(+)-1-(4-bromophenyl)ethylamine (20.52 g) and N-methylmorpholine (10.1 g) in toluene was added dropwise oxalyl chloride (6.5 g) with stirring, and the mixture was stirred for 1 hr. Water (50 mL) was added dropwise to the reaction mixture, and the precipitated crystals were collected by filtration and recrystallized from methanol to give (R,R)-N,N'-bis[1-(4-bromophenyl)ethyl]oxalylamide (18.26 g, 75%).
melting point: 280-281°C
IR(KBr, $cm^{-1}$): 3293, 1652, 1521
EI-MS: 454 ($M^+$)
Elemental analysis ($C_{18}H_{18}Br_2N_2O_2$) : Calculated [C:47.60, H:3.99, N:6.17], Found [C:47.70, H:7.05, N:6.16]
$^1$H-NMR (270MHz, DMSO-$d_6$) $\delta$: 1.42(6H,d,J=6.6Hz), 4.90-4.96(2H,m), 7.29 (4H,d,J=8.6Hz), 7.49 (4H,d,J=8.6H), 9.15(1H,s), 9.19(1H,s)
(2) To a borane-tetrahydrofuran complex (1 mol/L, 113 mL) was added (R,R)-N,N'-bis[1-(4-bromophenyl)ethyl] oxalylamide (18.26 g), and the mixture was stirred for 4 hr with reflux. Concentrated hydrochloric acid was added to the reaction mixture, and the mixture was further stirred for 30 min and cooled. The precipitated solid was filtered through celite, the filtrate was alkalified with sodium hydroxide and extracted with chloroform. The chloroform layer was washed with saturated brine, and dried over anhydrous sodium sulfate. Chloroform was evaporated under reduced pressure and the obtained oil was distilled under reduced pressure to give the titled compound (12.04 g, 70%).

boiling point: 156-158°C/0.02 mmHg
IR(neat, $cm^{-1}$) : 3308, 2961, 2925, 2851, 1590, 1486, 1404, 1120, 1070, 1009
$^1$HNMR (400MHz, DMSO-$d_6$) δ: 1.17(3H,s), 1.20(3H,s), 2.32(4H,q,J=8.6Hz), 2.6-3.5(2H,br), 7.34(4H,d,J=8.9Hz), 7.46(4H,d,J=8.6Hz)
EI-MS: 426($M^+$)
Elemental analysis ($C_{18}H_{22}Br_2N_2$): Calculated [C:50.73, H:5.20, N:6.57], Found [C:50.51, H:5.23, N:6.55]
$[\alpha]_D^{25}$ =+54 (c=1, $CH_3OH$).

**Production Example 2**

Methyl 4-(5-(imidazol-1-yl)-2-methylbenzoyl)-3,5-dimethylbenzoate

To a suspension of 4-(5-(imidazol-1-yl)-2-methylbenzoyl)-3,5-dimethylbenzoic acid (9.74 g) in methanol (100 mL) was added dropwise thionyl chloride (5.19 g), and the mixture was stirred at 50°C for 2 hr. The reaction mixture was concentrated under reduced pressure. The obtained residue was dissolved in ethyl acetate, washed with aqueous sodium hydrogencarbonate solution and dried over anhydrous sodium sulfate. Ethyl acetate was evaporated under reduced pressure and the obtained crystal was recrystallized from a ethyl acetate/n-heptane mixed solvent (1:3) to give the titled compound (7.40 g, 73%).
melting point: 164°C
IR(KBr, $cm^{-1}$) : 3120, 1716, 1686, 1510, 1434, 1311, 1236, 1222 $^1$H-NMR (270MHz, DMSO-$d_6$) δ: 2.15(6H,s), 2.56(3H, s), 3.87(3H,s), 7.05(1H,s), 7.33(1H,d,J=2.6Hz), 7.57(1H,s), 7.59(1H,d,J=8.6Hz), 7.74(2H,s), 7.82(1H,dd,J=2.6, 8.6Hz), 8.15(1H,s)
EI-MS: 348($M^+$)
Elemental analysis ($C_{21}H_{20}N_2O_3$) : Calculated [C:72.40, H:5.79, N:8.04], Found [C:72.45, H:5.82, N:8.01].

**Production Example 3**

4-(5-(imidazol-1-yl)-2-methylbenzoyl)-3,5-dimethylbenzoic acid isopropyl ester

In the same manner as in Production Example 2 except that 2-propanol was used instead of methanol, the title compound was obtained.
melting point: 164°C
IR(KBr, $cm^{-1}$): 3078, 1710, 1674, 1306, 1225
$^1$H-NMR(270MHz, DMSO-$d_6$)δ: 1.33 (6H,d,J=5.9Hz), 2.16(6H,s), 2.57(3H,s), 5.09-5.23(1H,m), 7.07(1H,s), 7.34(1H,d, J=2.6Hz), 7.58(1H,s), 7.60(1H,d,J=7.9Hz), 7.72(2H,s), 7.82(1H,dd,J=2.6,7.9Hz), 7.11(1H,s)
EI-MS: 376($M^+$)
Elemental analysis ($C_{23}H_{24}N_2O_3$) : Calculated [C:73.38, H:6.43, N:7.44], Found [C:73.49, H:6.45, N:7.42].

**Example 1**

Under an argon atmosphere, to a solution (2 mL) of (R,R)-N,N'-bis-(1-phenylethyl)ethane-1,2-diamine (0.77 g, 2.9 mmol) in toluene was added diethyl zinc (1 mol/L, 2.9 mL), and the mixture was stirred at room temperature for 30 min. To the reaction mixture was added a solution of 4-[5-(imidazol-1-yl)-2-methylbenzoyl]-3,5-dimethylbenzoic acid methyl ester (1.0 g, 2.9 mmol) in a toluene/N,N'-dimethylformamide mixed solvent (8:3) (10 mL), and the mixture was stirred for 10 min. Then, a solution of PMHS (1.12 g, 17 mmol) in toluene (3 mL) was added and the mixture was stirred for 4 hrs. To the reaction mixture was added 1 mol/L aqueous sodium hydroxide solution (4 mL) under ice-cooling and the mixture was stirred for 1 hr. Ethanol (18 mL) was then added and the mixture was stirred at 78°C for 8 hr. The reaction mixture was cooled and the solid was filtered through celite. The filtrate was adjusted to pH 5.0 with 1 mol/L hydrochloric acid and the precipitated crystals were collected by filtration to give compound 1 (0.66 g).
isolation yield: 68%
conversion: 100%
optical purity: 77%ee.

**Example 2**

Under an argon atmosphere, to a tetrahydrofuran solution (2 mL) containing molecular sieves MS3A (10 mg), (R,R)-N,N'-bis-(1-phenylethyl)ethane-1,2-diamine (0.27 g, 1 mmol) and ethylene glycol (0.06 g, 1 mmol) was added diethyl zinc (1 mol/L, 1 mL) and the mixture was stirred under ice-cooling for 10 min. A solution of 4-[5-(imidazol-1-yl)-2-methylbenzoyl]-3,5-dimethylbenzoic acid methyl ester (0.35 g, 1 mmol) in tetrahydrofuran (5 mL) was added to this reaction mixture, and the mixture was stirred for 20 min. Then, a solution of PMHS (0.39 g, 6 mmol) in tetrahydrofuran (2 mL) was added to the reaction mixture, and the mixture was warmed to room temperature and further stirred for 6 hr. To the reaction mixture was added 1 mol/L aqueous sodium hydroxide solution (4 mL) and the mixture was stirred for 1 hr under ice-cooling. Ethanol (18 mL) was then added and the mixture was further stirred at 78°C for 3 hr. The reaction mixture was cooled and the solid was filtered through celite. The filtrate was adjusted to pH 5.0 with 1 mol/L hydrochloric acid and the precipitated crystals were collected by filtration to give compound 1 (0.24 g).
isolation yield: 70%
conversion: 100%
optical purity: 84.2%ee.

**Example 3**

Under an argon atmosphere, to a tetrahydrofuran solution (2 mL) containing molecular sieves MS3A (10 mg), (R,R)-N,N'-bis-(1-phenylethyl)ethane-1,2-diamine (26.8 mg, 0.1 mmol) and ethylene glycol (6.2 mg, 0.1 mmol) was added diethyl zinc (1 mol/L, 0.1 mL) and the mixture was stirred under ice-cooling for 10 min. A solution of 4-[5-(imidazol-1-yl)-2-methylbenzoyl]-3,5-dimethylbenzoic acid methyl ester (0.35 g, 1 mmol) in tetrahydrofuran (5 mL) was added to this reaction mixture, and the mixture was stirred for 20 min. Then, a solution of PMHS (0.39 g, 6 mmol) in tetrahydrofuran (2 mL) was added to the reaction mixture, and the mixture was warmed to room temperature and further stirred for 6 hr. To the reaction mixture was added 1 mol/L aqueous sodium hydroxide solution (4 mL) and the mixture was stirred for 1 hr under ice-cooling. Ethanol (18 mL) was then added and the mixture was further stirred at 78°C for 3 hr. The reaction mixture was cooled and the solid was filtered through celite. The filtrate was adjusted to pH 5.0 with 1 mol/L hydrochloric acid and the precipitated crystals were collected by filtration to give compound 1 (0.23 g).
isolation yield: 68%
conversion: 100%
optical purity: 74.1%ee.

**Example 4**

Under an argon atmosphere, to a tetrahydrofuran solution (2 mL) containing molecular sieves MS3A (20 mg), (R,R)-N,N'-bis-(1-phenylethyl)ethane-1,2-diamine (71.4 mg,0.27 mol) and ethylene glycol (16.5 mg, 0.27 mol) was added diethyl zinc (1 mol/L, 0.27 mL) and the mixture was stirred under ice-cooling for 10 min. A solution of 4-[5-(imidazol-1-yl)-2-methylbenzoyl]-3,5-dimethylbenzoic acid isopropyl ester (1.0 g, 2.7 mmol) in tetrahydrofuran (5 mL) was added to this reaction mixture, and the mixture was stirred for 20 min. Then, a solution of PMHS (0.35 g, 5.4 mmol) in tetrahydrofuran (2 mL) was added to the reaction mixture, and the mixture was warmed to room temperature and further stirred for 24 hr. To the reaction mixture was added 1 mol/L aqueous sodium hydroxide solution (4 mL) and the mixture was stirred for 1 hr under ice-cooling. Ethanol (18 mL) was then added and the mixture was further stirred at 78°C for 3 hr. The reaction mixture was cooled and the solid was filtered through celite. The filtrate was adjusted to pH 5.0 with 1 mol/L hydrochloric acid and the precipitated crystals were collected by filtration to give compound 1 (0.76 g).
isolation yield: 85%
conversion: 100%
optical purity: 70.1%ee.

**Example 5**

Under an argon atmosphere, to a tetrahydrofuran solution (2 mL) containing molecular sieves MS3A (10 mg), (R,R)-N,N'-bis-[1-(4-bromophenyl)ethyl]ethane-1,2-diamine (85.2 mg, 0.20 mol) and ethylene glycol (12.4 mg, 0.20 mol) was added diethyl zinc (1 mol/L, 0.20 mL) and the mixture was stirred under ice-cooling for 10 min. A solution of 4-[5-(imidazol-1-yl)-2-methylbenzoyl]-3,5-dimethylbenzoic acid methyl ester (0.70 g, 2.0 mmol) in tetrahydrofuran (2 mL) was added to this reaction mixture, and the mixture was stirred for 20 min. Then, a solution of PMHS (0.26 g, 4.0 mmol) in tetrahydrofuran (1 mL) was added to the reaction mixture, and the mixture was warmed to room temperature and further stirred for 24 hr. A part of the reaction mixture was taken and 1 mol/L aqueous sodium hydroxide solution (4 mL) was added and the mixture was stirred for 1 hr under ice-cooling. Ethanol (18 mL) was added and the mixture was stirred at 78°C for 3 hr. The reaction mixture was cooled and the solid was filtered through celite. The filtrate was adjusted to pH 5.0 with 1 mol/L hydrochloric acid and ethyl acetate was added. The organic layer was analyzed by HPLC.
conversion: 26.8%
optical purity: 49.6%ee.

**Example 6**

Under an argon atmosphere, to a tetrahydrofuran solution (2 mL) containing molecular sieves MS3A (20 mg), (R,R)-N,N'-bis-[1-(4-bromophenyl)ethyl]ethane-1,2-diamine (0.13 g, 0.31 mol) and ethylene glycol (19.5 mg, 0.31 mol) was added diethyl zinc (1 mol/L, 0.31 mL) and the mixture was stirred under ice-cooling for 10 min. A solution (2 mL) of 4-[5-(imidazol-1-yl)-2-methylbenzoyl]-3,5-dimethylbenzoic acid methyl ester (0.11 g, 0.31 mmol) in tetrahydrofuran was added to this reaction mixture, and the mixture was stirred for 20 min. Then, a solution of PMHS (0.26 g, 4.0 mmol) in tetrahydrofuran (1 mL) was added to the reaction mixture, and the mixture was warmed to room temperature and further stirred for 10 hr. A part of the reaction mixture was taken and 1 mol/L aqueous sodium hydroxide solution (4 mL) was added thereto. The mixture was stirred for 1 hr under ice-cooling. Ethanol (18 mL) was then added and the mixture was further stirred at 78°C for 3 hr.
The reaction mixture was cooled and the solid was filtered through celite. The filtrate was adjusted to pH 5.0 with 1 mol/L hydrochloric acid and ethyl acetate was added. The organic layer was analyzed by HPLC.
conversion: 19.5%
optical purity: 78.7%ee.

**Comparative Example 1**

To a DMF solution (1 mL) of ruthenium (II) chloride benzene complex ($[Ru(C_6H_6)]_2Cl_2$) (10.3 mg) was added (S)-Xyl-BINAP (29.4 mg) under an argon atmosphere, which was prepared according to the method described in J. Org. Chem., 59, 3064-3076 (1994), and the mixture was stirred at 100°C for 10 min. The reaction mixture was concentrated under reduced pressure. To the obtained residue was added a solution (2 mL) of (-)-dpen (8.5 mg) in methylene chloride at room temperature and the mixture was stirred for 2 hr. The reaction mixture was concentrated under reduced pressure. To the obtained residue was added 2-propanol-tetrahydrofuran mixed solvent (1:2) (11.8 mL) solution consisting of 4-(5-(imidazol-1-yl)-2-methylbenzoyl)-3,5-dimethylbenzoic acid isopropyl ester (0.79 g) and a solution of potassium hydroxide in 2-propanol (1 mol/L, 0.1 mL), which was placed in an autoclave, and the mixture was stirred under a hydrogen atmosphere (3 MPa) at 40°C for 16 hr. The reaction mixture was filtered, and the filtrate was analyzed by HPLC. As a result, the conversion was 1.3%.

**Comparative Example 2**

In the same manner as in Example 1 except that 2,4,6,2'-tetramethylbenzophenone was used instead of 4-[5-(imidazol-1-yl)-2-methylbenzoyl]-3,5-dimethylbenzoic acid methyl ester, the corresponding benzhydrol compound was obtained.
isolation yield: 100%
conversion: not less than 99%
optical purity: 84%ee.

**Comparative Example 3**

In the same manner as in Comparative Example 1 except that 2,4,6,2'-tetramethylbenzophenone was used instead of 4-(5-(imidazol-1-yl)-2-methylbenzoyl)-3,5-dimethylbenzoic acid isopropyl ester, the corresponding benzhydrole compound was obtained. The conversion was 8.1%.

**Industrial Applicability**

According to the method of the present invention, an optically active 4-[hydroxy[5-(imidazol-1-yl)-2-methylphenyl]methyl]-3,5-dimethylbenzoic acid or an ester thereof can be produced high selectively in a high yield by an asymmetric reduction of a carbonyl group of 4-[5-(imidazol-1-yl)-2-methylbenzoyl]-3,5-dimethylbenzoic acid or an ester thereof.
This application is based on a patent application No. 2004-054928 filed in Japan on February 27, 2004, the contents of which are incorporated in full herein by this reference.

**Claims**

1. A method of preparing optically active 4-[hydroxy[5-(imidazol-1-yl)-2-methylphenyl]methyl]-3,5-dimethylbenzoic acid represented by the following formula (II)

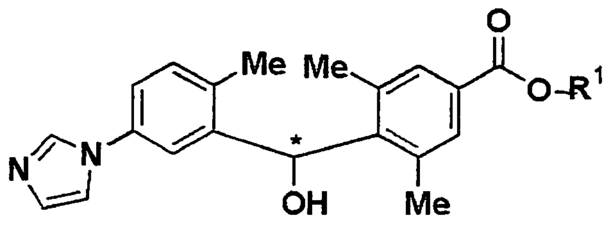

(II)

wherein $COOR^1$ is a carboxylic acid or a carboxylate and the carbon atom with * is an asymmetric carbon atom, or an ester thereof, from 4-[5-(imidazol-1-yl)-2-methylbenzoyl]-3,5-dimethylbenzoic acid represented by the following formula (I)

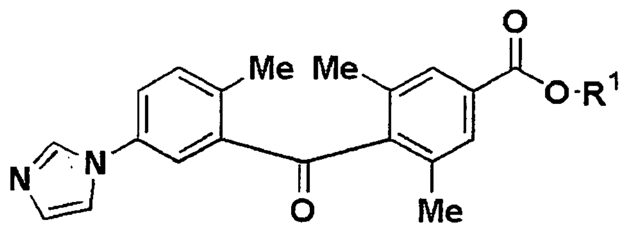

(I)

wherein $COOR^1$ is as defined above, or an ester thereof, which comprises reacting the compound represented by the formula (I) with a silane agent in the presence of a zinc compound represented by the following formula (III)

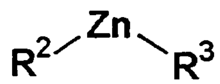

(III)

wherein $R^2$ and $R^3$ are each independently a lower alkyl group or a lower alkoxy group, or $R^2$ and $R^3$ in combination show an alkylenedioxy group optionally having substituent(s), and an optically active diamine compound represented by the following formula (IV)

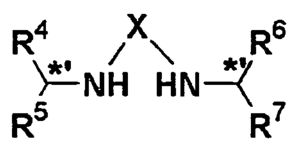

(IV)

wherein $R^4$ and $R^6$ are each independently a lower alkyl group, $R^5$ is an aryl group optionally having substituent(s), $R^7$ is an aryl group optionally having substituent(s) or a lower alkyl group, X is an alkylene group or a cycloalkylene group, and one or both of the two carbon atoms with *' is(are) asymmetric carbon atom(s).

**2.** The method of claim 1, wherein $R^1$ is an alkyl group optionally having substituent(s), a cycloalkyl group optionally having substituent(s) or an aralkyl group optionally having substituent(s) on the ring and/or the chain.

**3.** The method of claim 1 or 2, wherein $R^2$ and $R^3$ are each independently a lower alkyl group.

**4.** The method of claim 3, wherein the reaction is carried out in the presence of alcohol or glycol.

**5.** The method of any one of claims 1 to 4, wherein the optically active diamine compound is a N,N'-bis-(1-phenylethyl) ethane-1,2-diamine compound represented by the following formula (V)

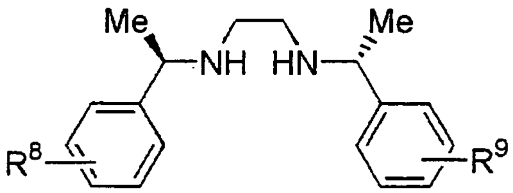 (V)

wherein $R^8$ and $R^9$ are each independently a hydrogen atom, a halogen atom, a lower alkyl group, a lower alkoxy group, a nitro group, a cyano group or an aryl group optionally having substituent(s),
or an optical isomer thereof.

**6.** The method of claim 5, wherein the N,N'-bis-(1-phenylethyl)ethane-1,2-diamine compound is (R,R)-N,N'-bis-(1-phenylethyl)ethane-1,2-diamine or (R,R)-N,N'-bis-[1-(4-bromophenyl)ethyl]ethane-1,2-diamine.

**7.** The method of any one of claims 1 to 6, wherein the silane agent is selected from the group consisting of trimethylsilane, diethylsilane, triethylsilane, phenylsilane, diphenylsilane, methylphenylsilane, dimethylphenylsilane, diethylphenylsilane, methyldiphenylsilane, tert-butyldimethylsilane, tert-butyldiphenylsilane, trimethoxysilane, diethoxysilane, triethoxysilane, tributoxysilane, triphenoxysilane, (trimethylsiloxy)dimethylsilane, bis(trimethylsiloxy) methylsilane, triisopropoxysilane, tris(trimethylsiloxy)silane, tris(trimethylsilyl)silane and polymethylhydrosiloxane.

**8.** A method of preparing an optically active 4-[hydroxy[5-(imidazol-1-yl)-2-methylphetyllmethyl]-3,5-dimethylbenzoic acid ester which comprises reacting 4-[5-(imidazol-1-yl)-2-methylbenzoyl]-3,5-dimethylbenzoic acid ester with polymethylhydrosiloxane in the presence of zinc di-lower alkyl and an optically active diamine compound represented by the formula (V).

**9.** The method of claim 8 further comprising a reaction in the presence of alcohol or glycol.

**10.** The method of claim 9 further comprising a reaction in the presence of ether.

## INTERNATIONAL SEARCH REPORT

International application No.
PCT/JP2005/003107

A. CLASSIFICATION OF SUBJECT MATTER
Int.Cl[7] C07D233/60, C07B53/00//C07B61/00

According to International Patent Classification (IPC) or to both national classification and IPC

B. FIELDS SEARCHED

Minimum documentation searched (classification system followed by classification symbols)
Int.Cl[7] C07D233/60, C07B53/00//C07B61/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
CA(STN), CAPLUS(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | JP 61-277670 A (Yoshitomi Pharmaceutical Industries, Ltd.), 08 December, 1986 (08.12.86), & US 4661603 A | 1-10 |
| A | JP 61-282366 A (Yoshitomi Pharmaceutical Industries, Ltd.), 12 December, 1986 (12.12.86), (Family: none) | 1-10 |
| A | JP 2000-290259 A (WELFIDE CORP.), 17 October, 2000 (17.10.00), & WO 98/32740 A1 & EP 972768 A1 & US 6066739 A | 1-10 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>14 April, 2005 (14.04.05) | Date of mailing of the international search report<br>10 May, 2005 (10.05.05) |
| Name and mailing address of the ISA/<br>Japanese Patent Office<br>Facsimile No. | Authorized officer<br><br>Telephone No. |

Form PCT/ISA/210 (second sheet) (January 2004)

**INTERNATIONAL SEARCH REPORT**

International application No.
PCT/JP2005/003107

C (Continuation). DOCUMENTS CONSIDERED TO BE RELEVANT

| Category* | Citation of document, with indication, where appropriate, of the relevant passages | Relevant to claim No. |
|---|---|---|
| A | JP 2-215771 A (Yoshitomi Pharmaceutical Industries, Ltd.), 28 August, 1990 (28.08.90), (Family: none) | 1-10 |
| A | JP 2003-221334 A (Mitsubishi Pharma Corp.), 05 August, 2003 (05.08.03), & WO 97/24333 A1 & EP 881218 A1 & US 6258834 B1 | 1-10 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9724333 A
- JP 2000290259 A
- JP 2001515875 A
- JP 2004054928 A


### Non-patent literature cited in the description


- *J. Am. Chem. Soc.,* 1996, vol. 118, 2521-2522
- *Org. Lett.,* 2000, vol. 2, 659-662
- *Angewandte Chemie International Edition in English,* 1998, vol. 37, 1986-2012
- *Synthesis,* 1974, 633-651
- *J. Am. Chem. Soc.,* 1999, vol. 121, 9473-9474
- *Journal of Organometallic Chemistry,* 2001, vol. 624, 367-371
- *J. Am. Chem. Soc.,* 2001, vol. 123, 12917-12918
- *J. Am. Chem. Soc.,* 1999, vol. 121, 6156-6166
- *J. Org. Chem.,* 1999, vol. 64, 2582-2589
- *Tetrahedron,* 2004, vol. 60, 1781-1789
- *Tetrahedron: Asymmetry,* 2003, vol. 14, 3937-3941
- *Tetrahedron: Asymmetry,* 1994, vol. 5, 699-708
- *J. Am. Chem. Soc.,* 1999, vol. 121, 6158-6166
- *J. C. S. Chem. Comm.,* 1987, 1409-1410
- *J. Org. Chem.,* 1994, vol. 59, 3064-3076